# EUROPÄISCHE PATENTSCHRIFT

(11) **EP 1 466 639 B1**
(45) Veröffentlichungstag und Bekanntmachung des Hinweises auf die Patenterteilung: **31.05.2006**
(21) Anmeldenummer: 03029820.2
(22) Anmeldetag: 23.12.2003
(51) Int. Cl.: A61M 5/31, A61M 5/315

(54) **Spritzenzylinder sowie Spritze mit diesem Spritzenzylinder**
Syringe barrel and syringe including such syringe barrel
Cylindre de seringue et seringue contenant ce cylindre

(30) Priorität: 11.04.2003 DE 20305913 U
(43) Veröffentlichungstag der Anmeldung: 13.10.2004
(73) Patentinhaber: Henke-Sass, Wolf GmbH, 78507 Tuttlingen (DE)
(72) Erfinder: Schulz, Dieter, 78570 Mühlheim a.D. (DE)
(74) Vertreter: Geyer, Fehners & Partner

(56) Entgegenhaltungen:
- EP-A- 0 825 223
- WO-A-01/02454
- WO-A-99/17833
- DE-A- 3 408 618
- US-A- 4 557 959
- US-A- 6 007 520
- US-A1- 2003 032 923
- US-B1- 6 210 359

## Beschreibung

Die Erfindung bezieht sich auf einen Spritzenzylinder gemäß dem Oberbegriff des Anspruch 1 für eine veterinär- oder humanmedizinische Spritze, wobei der Spritzenzylinder zur Aufnahme eines Kolbens, der auf eine zu injizierende Flüssigkeit drückt, vorgesehen ist und eine Wandung aufweist. Die Erfindung bezieht sich weiter auf eine Spritze mit einem solchen Spritzenzylinder, wie or z.B. in der US-A-6007520 offenbart ist.

Bei veterinär- oder humanmedizinischen Spritzen sind die Spritzenzylinder üblicherweise transparent, so daß man bei der Verwendung sehen kann ob bzw. wie viel Flüssigkeit in der Spritze aufgezogen ist. Der einfachen Herstellbarkeit sind Spritzenzylinder dabei meist aus einem einzigen Material gefertigt, üblicherweise durch Spritzguß aus Kunststoff.

Insbesondere bei wiederverwendbaren Spritzen werden hohe Anforderungen an die Robustheit gestellt. Dies gilt in verstärktem Maße für veterinärmedizinische Spritzen, die aus einem größeren Volumen des Spritzenzylinders mit einer Betätigung eine bestimmte Einzel-Dosis abzugeben in der Lage sind, sogenannte Repetier-Spritzen.

Bei solchen Spritzen besteht je nach Anwendungfail mitunter der Bedarf, einen möglichst großvolumigen Spritzenzylinder verwenden zu können, um nach einem einzigen Aufziehvorgang möglichst viele Einzelinjektionen durchführen zu können. Dann muß die aufgezogene Flüssigkeit, beispielsweise ein Medikament oder ein sonstiges zu verabreichendes Präparat, es erlauben, in einem großen Volumen aufgezogen und über einen längeren Zeitraum in Einzelinjektionen verteilt zu werden.

Es ist Aufgabe der Erfindung, einen Spritzenzylinder sowie eine Spritze bereitzustellen, mit denen man auch größere Mengen an in Injektionen abzugebender Flüssigkeit über einen längeren Zeitraum vorhalten kann.

Diese Aufgabe wird erfindungsgemäß mit einem Spritzenzylinder gemäß dem Anspruch 1 gelöst. Die Aufgabe wird weiter gelöst durch eine Spritze mit einem solchen Spritzenzylinder gemäß dem Anspruch 7.

Die erfindungsgemäße Ausgestaltung des Spritzenzylinders schützt einmal aufgezogene Flüssigkeit vor schädigender UV-Strahlung, so daß dadurch eine längere Standzeit einmal aufgezogener Flüssigkeit gegeben ist. Dies ist insbesondere vorteilhaft, wenn eine mit dem erfindungsgemäßen Spritzenzylinder ausgerüstete Spritze im Freien verwendet werden soll, wie es in der Veterinärmedizin oftmals der Fall ist. Massenapplikationen von Flüssigkeiten, beispielsweise Massenimpfungen von Schafen, Schweinen oder Rindern, sind nun im freien Feld auch bei starker Sonneneinstrahlung möglich, ohne daß eine Schädigung aufgezogener Flüssigkeit zu befürchten wäre.

Um sicher erkennen zu können, ob bzw. wie viel Flüssigkeit in den Spritzenzylinder eingebracht wurde, ist eine Ausführungsform vorteilhaft, bei der die Wandung des Spritzenzylinders für Licht (also sichtbare Strahlung) transparent ist, wenn auch nicht mehr unbedingt farblos. Um die eigens erwähnten durchsichtigen Eigenschaften dabei zu erhalten, ist das Material der Wandung des Spritzenzylinders so gewählt, daß es spektral selektiv absorbiert, d.h. UV-Strahlung absorbiert, dagegen sichtbare Strahlung, d.h. Licht, möglichst transmittiert. Oftmals wird sich beim Anstreben dieses Ziels eine bernsteinfarbene Färbung des Spritzenzylinders einstellen.

Für einen besonders guten Schutz aufgezogener Flüssigkeit vor schädigender UV-Strahlung ist hat die Wandung des Spritzenzylinders bei einer Wellenlänge zwischen 350 und 380 oder vorteilhafterweise auch 400 nm, vorteilhafterweise sogar in dem gesamten Bereich, einen Absorptionsgrad von mindestens 20% oder höher hat. Ein Wert von 30 % ist besonders bevorzugt, da er einen besseren Schutz bietet. Ein nochmals gesteigerter Absorptionsgrad von 40 oder 50 % verbessert den Schutz vor schädigender UV-Strahlung weiter.

Unter dem Gesichtspunkt der Herstellbarkeit ist ein Spritzenzylinder, der durch Spritzguß gefertigt werden kann, günstig. Als Material für den Spritzenzylinder ist deshalb spritzgußfähiger Kunststoff zu bevorzugen. Eine Klasse von Kunststoffen, die einerseits spritzgußfähig sind und andererseits die gewünschten UV-Absorptionseigenschaften aufweisen, ist Polyphenolsulfon. Es ist deshalb zu bevorzugen, daß der Spritzenzylinder dieses Material aufweist oder sogar vollständig daraus, z.B. durch Spritzguß, gefertigt ist, zumindest in Bereich der Wandung. Natürlich kommen auch andere Materialien in Frage, wie beispielsweise Glas oder Polycarbonat, die zwar von Haus aus klar transparent sind, jedoch durch Zugabe entsprechender Substanzen mit den gewünschten UV-strahlungsabsorbierenden oder - reflektierenden Eigenschaften versehen werden können.

Polyphenolsulfon hat sich weiter als vorteilhaft herausgestellt, da es zum einen sehr stabil gegenüber chemisch aggressiven Substanzen oder Säuren, beispielsweise Vitamin A-Säure, ist. Zum anderen ist das Schrumpfverhalten von Polyphenolsulfon bei der Herstellung nahezu identisch mit den Eigenschaften von Polycarbonat, so daß die Herstellung eines Spritzenzylinders, der bislang aus Polycarbonat gefertigt wurde, einfach auf Polyphenolsulfon umgestellt werden kann, ohne daß Spritzgußwerkzeuge oder Sollmaße abgeändert werden müßten.

UV-Strahlung kann in den Spritzenzylinder aufgezogene Flüssigkeit vor allem durch die Wandung des Spritzenzylinders hindurch erreichen. Zwar ist es möglich und aus Kostengesichtspunkte manchmal auch günstig, den gesamten Spritzenzylinder inklusive Injektionsnadelansatz aus demselben Material wie die Wandung zu fertigen, so daß der Spritzenzylinder insgesamt UV-strahlungsabsorbierend oder -reflektierende Eigenschaften hat, jedoch ist es aus mechanischen Gründen oftmals zu bevorzugen, eine Aufnahme für eine Injektionsspritze aus anderem Material herzustellen. Dies gilt insbesondere für Injektionsspritzen, die mit genormten oder weit verbreiteten Injektionsnadel-Verriegelungsmechanismen, z.B. einem Mechanismus nach Luer-Lock, versehen sind. Hierfür sind insbesondere bei wiederverwendbaren Spritzen metallische Aufnahmen der Stabilität wegen zu bevorzugen. Es ist deshalb in einer Ausführungsform der Erfindung bevorzugt, daß der Spritzenzylinder an einem Ende eine Aufnahme für eine Injektionsnadel aufweist.

Am anderen Ende kann der Spritzenzylinder in eine Handhabungseinheit, die zum Halten, Abstützen oder Betätigen der Spritze dient, eingesetzt sein, insbesondere über eine Schraubverbindung. Ein solcher Spritzenzylinder hat auch den Vorteil, daß mit herkömmlichen Spritzenzylindern ausgerüstete Spritzen entsprechend nachgerüstet werden können.

Natürlich kommt der erfindungsgemäße Spritzenzylinder insbesondere auch für Wegwerfspritzen in Frage. Dann werden vorteilhafterweise eine Spitze zum Aufsetzen einer Injektionsnadel sowie entsprechende Handhabungselemente, beispielsweise Griffe, direkt am Spritzenzylinder angeformt.

Die Erfindung wird nachfolgend anhand der Zeichnungen beispielshalber noch näher erläutert. In den Zeichnungen zeigt:
- Fig. 1: eine perspektivische Ansicht einer Veterinärspritze,
- Fig. 2: einen vertikalen Längsschnitt durch die Veterinärspritze und
- Fig. 3: spektrale Absorptionskurven des Materials des Spritzenzylinders der Veterinärspritze der Fig. 1.

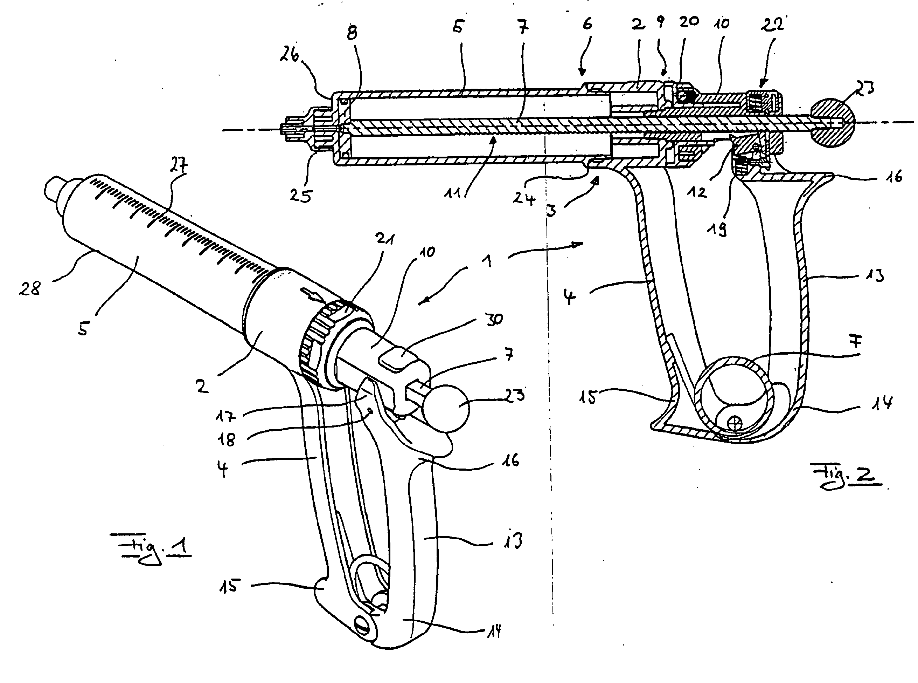

in Fig. 1 ist eine Veterinärspritze 1 gezeigt, die umfaßt einen Grundkörper 2, an dessen Unterseite 3 ein Handgriff 4 angeordnet ist, einen Spritzenzylinder 5, der an der Vorderseite 6 des Grundkörpers 2 eingesetzt ist und in den eine Kolbenstange 7 mit einem Kolben 8 einsteht. Die Kolbenstange 7 ist in einem an der Rückseite 9 des Grundkörpers 2 angeordneten Führungskörper 10 längsverschieblich gehalten. Die Kolbenstange 7 ist an ihrer Unterseite als Zahnstange 11 ausgebildet, in die eine Klinke 12 einrasten kann, die von einem Bedienungshebel 13 antreibbar ist. Der Bedienungshebel 13 ist mit einem ersten Ende 14 am freien Ende 15 des Handgriffs 4 schwenkbar befestigt und mit seinem zweiten Ende 16 an dem Führungskörper 10 in Längsrichtung der Veterinärspritze 1 geführt. Eine Feder F spannt den Bedienungshebel 13 vom Handgriff 4 weg. Das zweite Ende 16 des Bedienungshebels 13 ist im Bereich des Führungskörpers 10 als Gabel 17 ausgebildet, die den Führungskörper 10 seitlich umgreift. In dieser Gabel 17 ist eine horizontal und quer zur Bewegungsrichtung der Kolbenstange 7 gelagerte Achse 18 vorgesehen, auf der die Klinke 12 schwenkbar gelagert ist und unter von einer in dem Bedienungshebel 13 vorgesehenen Feder 19 gegen die Zahnstange 11 gedrückt wird.

Wird der Bedienungshebel 13 nach vorn in Richtung auf den Handgriff 4 bis zu einem Anschlag bewegt, wird die Kolbenstange 7 in den Spritzenzylinder 5 hineinbewegt. Der Bewegungsweg des Bedienungshebels 13 kann dabei über eine Anschlagbegrenzungsvorrichtung 20 eingestellt werden, so daß die Ausspritzmenge dosiert werden kann. Die Anschlagbegrenzungsvorrichtung 20 ist in den Führungskörper 10 integriert und weist einen zwischen der Rückseite 9 des Grundkörpers 2 einerseits und dem Führungskörper 10 andererseits angeordneten und verdrehbaren Einstellring 21 auf, der auf einzelne Dosierstufen eingestellt werden kann und mittels einer geeigneten Mechanik den Bewegungsweg des Bedienungshebels 13 für jede Betätigung begrenzt.

Am hinteren Ende des Führungskörpers 10 ist eine Verriegelungsvorrichtung 22 zum Verriegeln der Zahnstange 7 gegen ein unbeabsichtigtes Zurückziehen der Kolbenstange 7 vorgesehen. Weiter ist am Ende der Kolbenstange 7 ein Knopf 23 vorgesehen, mit dem der Kolben 8 wieder in seine Ausgangsstellung bewegt und damit auch der Spritzenzylinder 5 wieder mit befüllt werden kann.

Der Spritzenzylinder 5 ist über eine Schraubverbindung 24 in den Handgriff 4 abdichtend eingesetzt, so daß der Vorschub des Kolbens 8, der auf in den Spritzenzylinder 5 aufgezogene Flüssigkeit drückt, durch dichtende Anlage an der Innenseite des Spritzenzylinders 5 die Flüssigkeit an der Spritze ausstößt. Am injektionsnadelseitigen Ende 26 verfügt der Spritzenzylinder 5 über eine geeignete Aufnahme 25 für eine Injektionsnadel.

Der Spritzenzylinder 5 ist für Licht geeignet transparent, so daß mittels einer Skalierung 27, die an der Wandung 28 des Spritzenzylinders 5 gebildet ist, einfach überprüft werden kann, welche Menge an Flüssigkeit in die Spritze aufgezogen wurde.

Im übrigen weist die Wandung 28 des Spritzenzylinders 5 UV-strahlungsabsorbierende Eigenschaften auf, so daß in den Spritzenzylinder 5 aufgezogene Flüssigkeit vor schädigenden Einflüssen von UV-Strahlung geschützt ist.

Die UV-strahlungsabsorbierenden Eigenschaften der Wandung 28 sind dadurch erreicht, daß der Spritzenzylinder 5 aus Polyphenolsulfon hergestellt ist. Dazu wird in der beschriebenen Ausführung das Material Radel R-5100 MT15 der Solvay Advanced Polymers, Alpharetta, Georgia, USA, verwendet, das ein spritzgußfähiges bernsteinfarbenes Polyphenylsulfonharz ist. Dieses Material wurde vor dem Spritzguß 2,5 Std. bei etwa 150°C getrocknet und dann bei einer Temperatur von 360 bis 390° in eine Form spritzgegossen, wobei die Temperatur der Form zwischen 140 und 165° lag.

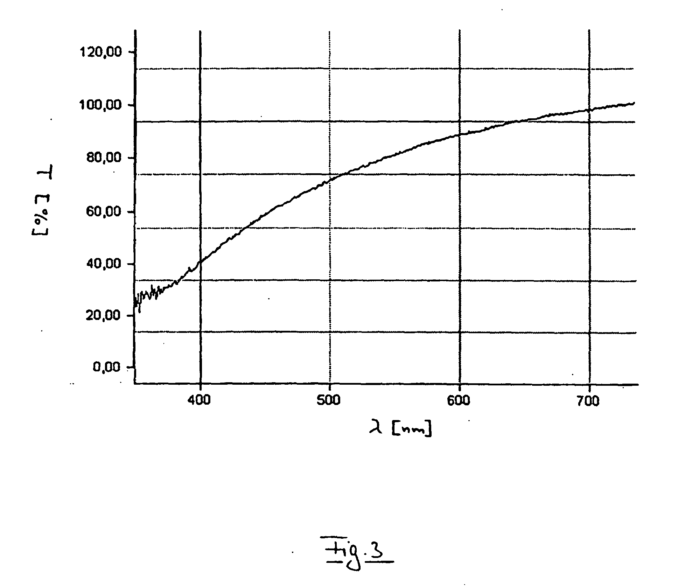

Fig. 3 zeigt die Transmissionseigenschaften der Wandung 28 des Spritzenzylinders 5, wobei ein in Prozent angegebene Transmissionsgrad T als Funktion der in nm angegebenen Wellenlänge λ aufgetragen ist. Wie dort zu sehen ist, hat die Wandung in einem Wellenlängenbereich zwischen 350 und 380 nm einen Absorptionsgrad von über 60 %. Mit ansteigender Wellenlänge sinkt der Absorptionsgrad, d.h. der Transmissionsgrad steigt. Bei einer Wellenlänge von 400 nm wird ein Transmissionsgrad von etwa 40 % überschritten. Der in Fig. 3 dargestellte Transmissionsgradverlauf der Wandung des Spritzenzylinders führt zur Bernsteinfarbe des Zylinders. Natürlich sind andere Färbungen durch Beigabe von Farbstoffen möglich, beispielsweise kann durch Zugabe eines dunklen Farbstoffs in der Konzentration von etwa 3 % ein grauer Spritzenzylinder erreicht werden.

Die Schrumpfung nach dem Abformen liegt bei 0,7 % und ist somit identisch mit Polycarbonat. Für die Herstellung des Spritzenzylinders 5 werden deshalb Spritzgußformen verwendet, die für Polycarbonatspritzenzylinder entworfen wurden. Durch das Herstellen des Spritzenzylinders mit einem Spritzgußverfahren besteht der gesamte Spritzenzylinder 5 aus dem spritzbaren Kunststoff, so daß die UV-strahlungsabsorbierenden Eigenschaften im Bereich des gesamten Spritzenzylinders vorliegen.

Der Knopf 23 der Spritze der Fig. 1 und 2 ist auf das Ende der Kolbenstange 11 lösbar aufgesetzt, im Beispiel der Fig. 2 ist eine Schraubverbindung verwendet. Er kann somit einfach ausgetauscht werden. Für die Spritze sind Knöpfe verschiedener Farben vorgesehen, so daß eine einfache Unterscheidung äußerlich ansonsten gleicher Spritzen für unterschiedliche Anwendungen möglich ist. Der Anwender kann Spritzen mit unterschiedlichen Flüssigkeiten, beispielsweise Medikamenten, aufziehen und jeder Flüssigkeit einen andersfarbigen Knopf 23 zuordnen. Dies ist besonders vorteilhaft, da durch den UV-strahlungsabsorbierenden Spritzenzylinder 5 es nunmehr möglich ist, mehrere Flüssigkeiten aufzuziehen und derart vorbereitete Spritzen über einen längeren Zeitraum zu verwenden. Die Unterscheidung durch unterschiedlich farbige Knöpfe 23 verhindert hier die Gefahr von Verwechslungen.

Durch die Schraubverbindung 24 zwischen Spritzenzylinder 5 und Handgriff 4 kann der Spritzenzylinder zur Reinigung einfach vom Handgriff abgenommen werden. Im Zusammenhang mit dem thermisch sehr stabilen Polyphenolsulfon-Material des Spritzenzylinders 5 ist eine volle Autoklavierbarkeit erreicht.

## Patentansprüche

1. Spritzenzylinder für eine veterinär- oder humanmedizinische Spritze (1) und zur Aufnahme eines Kolbens (8) der auf eine zu injizierende Flüssigkeit drückt, wobei der Spritzenzylinder eine Wandung (28) aufweist ; wobei das Material der Wandung UV-strahlungsabsorbierende oder -reflektierende Eigenschaften hat, **dadurch gekennzeichnet, daß** das Material der Wandung UV-Strahlung spektral selektiv absorbiert und bei einer wellenlänge zwischen 350 und 380 nm einen Absorptions = grad von mindestens 20% hat.

2. Spritzenzylinder nach Anspruch 1, **dadurch gekennzeichnet, daß** die Wandung (28) bei einer Wellenlänge zwischen 350 und 380 nm einen Absorptionsgrad von mindestens 30 % hat.

3. Spritzenzylinder nach Anspruch 2, **dadurch gekennzeichnet, daß** die Wandung (28) bei einer Wellenlänge zwischen 350 und 380 nm einen Absorptionsgrad von mindestens 40 %, vorzugsweise 50 % hat.

4. Spritzenzylinder nach einem der obigen Ansprüche, **dadurch gekennzeichnet, daß** das Material Polyphenolsulfon aufweist.

5. Spritzenzylinder nach einem der obigen Ansprüche, **dadurch gekennzeichnet, daß** der Spritzenzylinder (5) aus einem spritzbaren Kunststoff besteht.

6. Spritzenzylinder nach einem der obigen Ansprüche, **dadurch gekennzeichnet, daß** der Spritzenzylinder (5) an einem Ende eine Aufnahme (25) für eine Injektionsnadel aufweist.

7. Veterinär- oder humanmedizinische Spritze zur Injektion von Flüssigkeiten, mit einem Spritzenzylinder (5) nach einem der obigen Ansprüche.

8. Spritze nach Anspruch 7, **dadurch gekennzeichnet, daß** der Spritzenzylinder (5) in eine Handhabungseinheit (3) zum Halten, Abstützen oder Betätigen der Spritze (1) eingesetzt, insbesondere eingeschraubt ist.

9. Spritze nach Anspruch 7, **dadurch gekennzeichnet, daß** sie als Wegwerfspritze ausgebildet ist.

## Claims

1. Syringe barrel for a syringe (1) for veterinary or human medicine and for receiving a piston (8) pressing on a liquid to be injected, said syringe barrel comprising a wall (28), the material of said wall having UV radiation-absorbing or UV radiation-reflecting properties, **characterised in that** the material of the wall absorbs UV radiation in a spectrally selective manner and has a degree of absorption of at least 20% at a wavelength of from 350 to 380 nm.

2. Syringe barrel according to claim 1, **characterised in that** the wall (28) has a degree of absorption of at least 30% at a wavelength of from 350 to 380 nm.

3. Syringe barrel according to claim 2, **characterised in that** the wall (28) has a degree of absorption of at least 40 %, preferably 50%, at a wavelength of from 350 to 380 nm.

4. Syringe barrel according to any one of the above claims, **characterised in that** the material comprises polyphenol sulfone.

5. Syringe barrel according to any one of the above claims, **characterised in that** the syringe barrel (5) consists of an injection-mouldable plastic material.

6. Syringe barrel according to any one of the above claims, **characterised in that** the syringe barrel (5) comprises, at one end thereof, a receiving part (25) for an injection needle.

7. Syringe for veterinary or human medicine for injection of liquids, which comprises a syringe barrel (5) according to any one of the above claims.

8. Syringe according to claim 7, **characterised in that** the syringe barrel (5) is inserted, in particular screwed, into a manipulating unit (3) for holding, supporting or actuating the syringe (1).

9. Syringe according to claim 7, **characterised in that** it is provided as a disposable syringe.

## Revendications

1. Cylindre de seringue pour une seringue (1) du domaine de la médecine vétérinaire ou humaine et destiné à recevoir un piston (8) qui presse sur un liquide à injecter, le cylindre de la seringue présentant une paroi (28) et le matériau de la paroi possédant des propriétés d'absorption ou de réflexion des rayons UV, **caractérisé en ce que** le matériau de la paroi absorbe de manière sélective le spectre du rayonnement UV et présente un degré d'absorption d'au moins 20 % pour une longueur d'onde comprise entre 350 et 380 nm.

2. Cylindre de seringue selon la revendication 1, **caractérisé en ce que** pour une longueur d'onde comprise entre 350 et 380 nm, la paroi présente un degré d'absorption d'au moins 30 %.

3. Cylindre de seringue selon la revendication 2, **caractérisé en ce que** pour une longueur d'onde comprise entre 350 et 380 nm, la paroi (28) présente un degré d'absorption d'au moins 40 %, de préférence de 50%.

4. Cylindre de seringue selon l'une des revendications précédentes, **caractérisé en ce que** le matériau contient du polyphénolsulfone.

5. Cylindre de seringue selon l'une des revendications précédentes, **caractérisé en ce que** le cylindre (5) de la seringue est constitué d'une matière plastique injectable.

6. Cylindre de seringue selon l'une des revendications précédentes, **caractérisé en ce que** le cylindre (5) de la seringue présente, à une extrémité, un embout (25) pour une aiguille d'injection.

7. Seringue médicale vétérinaire ou humaine pour l'injection de liquides, comportant un cylindre (5) selon l'une des revendications ci-dessus.

8. Seringue selon la revendication 7, **caractérisé en ce que** le cylindre (5) de la seringue est inséré, en particulier vissé, dans une unité de manipulation (3) pour tenir, soutenir ou actionner la seringue (1).

9. Seringue selon la revendication 7, **caractérisé en ce qu'**elle est réalisée sous la forme d'une seringue jetable.
